# EUROPEAN PATENT APPLICATION

(11) **EP 1 375 673 A1**
(43) Date of publication of application: **02.01.2004**
(21) Application number: 02705325.5
(22) Date of filing: 19.03.2002
(51) Int. Cl.: C12Q 1/68, C12M 1/00

(54) **GENE ANALYSIS METHOD AND ANALYZER THEREFOR**

(30) Priority: 29.03.2001 JP 2001095419; 08.03.2002 JP 2002063943
(71) Applicant: Japan Science and Technology Corporation, Kawaguchi-shi, Saitama-ken 332-0012 (JP); Gl Sciences Incorporated, Tokyo 163-1130 (JP)
(72) Inventor: KONDO, Toshihiko, Maebashi-shi, Gunma 371-0037 (JP); ABE, Shuzo c/o GL Sciences Inc. Technical Center, Iruma-shi, Saitama 358-0032 (JP); SHIKAMA, Nobuyosi c/o GL Sciences Inc. Techn.Ctr., Iruma-shi, Saitama 358-0032 (JP); SHINTANI, Yukihiro c/o GL Sciences Inc.Techn.Ctr., Iruma-shi, Saitama 358-0032 (JP)
(74) Representative: Schmid, Wolfgang
(86) International application number: PCT/JP2002/002583
(87) International publication number: WO 2002/079510

(57) **Abstract**

This invention relates to a method for analyzing a gene and an analysis device thereof which are suited to be used, for example, as a fully automatic gene analysis device or a fully automatic gene diagnostic device which can be simplified in structure, made compact in size and light in weight, and manufactured at a low cost and in which detection of various kinds of DNA can be conducted rapidly and with precision and analysis of a plurality of genes can be made simultaneously from different kinds of samples by restraining, as much as possible, the use of a valve in an analytic system, thereby preventing admixture of cross contamination.

It relates to a gene analysis method comprising the steps of extracting a target nucleic acid from a biological sample (23) and amplifying a target DNA, thereafter, introducing a reaction eluent (L) containing a predetermined DNA into a stationary-phase DNA probe (53) having a predetermined temperature and arranged in series or in parallel and separating a DNA complementary to the stationary-phase DNA probe (53).

The above-mentioned gene analysis method comprises a plurality of stationary-phase DNA probes (64 through 66) at least a part of which can be set to a temperature for forming a double strand of DNA to be tested and a reaction eluent containing the same or different kinds of DNA which have been amplified, is introduced into the stationary-phase DNA probes (64 through 66).

## Description

### Technical Field

This invention relates to a method for analyzing a gene and an analysis device thereof which are suited to be used, for example, as a fully automatic gene analysis device or a fully automatic gene diagnostic device which can be simplified in structure, made compact in size and light in weight, and manufactured at a low cost and in which detection of various kinds of DNA can be conducted rapidly and with precision and analysis of a plurality of genes can be made simultaneously from different kinds of samples by restraining, as much as possible, the use of valves in an analytic system, thereby preventing admixture of cross contamination.

### Background Art

Recently, the technique for analyzing genetic data of deoxyribonucleic acid (DNA) which carries genetic data, has been progressed remarkably and the use of this technique in the fields of gene diagnosis and medical treatment is expected.

The analytic technique of a genetic data includes a DNA extraction process for collecting DNA of a biological sample such as blood, cells and the like and purifying the same, a DNA amplification process for extensively increasing the amount of a target DNA, a DNA detection process for separating the component of DNA and detecting the same, and an analysis process for analyzing the resultant. Those main processes are carried out by a gene analysis device sequentially and consistently.

A gene analysis apparatus disclosed in Japanese Patent Application Laid-Open No. H06-327476, for example, comprises a dispenser element, a container conveying element, a centrifugal separation element, a mixing element, a container inverting element, a heating/drying element, and a gel electrophoresis element. The motion of those component elements is controlled by a computer and the sequence of treatment processes is fully automated.

However, since this conventional apparatus needs , in addition to the need of the centrifugal separation element and the gel electrophoresis element, various mechanisms controlled by program when a sample eluent is moved in accordance with each process, the apparatus becomes complicated, and large both in size and in weight, thus resulting in high cost. Moreover, since various valves are placed in the passageway of the sample eluent, the problem of cross contamination arises, thus resulting in less reliability of analysis precision.

Moreover, there are such problems that the electrophoresis requires much time for separation and the separation precision is generally not good. The similar problems are also common to the gene analysis apparatus disclosed in Japanese Patent Application Laid-Open No. H07-75544.

As one technique capable of solving those problems, a gene analysis apparatus disclosed in Japanese Patent Application Laid-Open No. H10-239300, for example, is developed under a base sequence specific thermal elution chromatographic method (SSTEC method), in which DNA, after being subjected to PCR amplification, is injected into a stationary-phase DNA column, then the DNA, which is complementary to the stationary-phase DNA, is trapped, so that the trapped DNA is separated for analysis from the base sequence which is partly not complementary to the stationary-phase DNA due to difference in melting temperature by increasing in temperature of the column while supplying an eluent.

The gene analysis apparatus in accordance with the SSTEC method comprises an automatic sampler unit for conducting a sequence of operations from the preparation of gene samples to the column injection, a feed eluent gradient unit for feeding at least a buffer eluent and a reaction eluent, a thermal controller unit including a column filled with a stationary-phase DNA probe, a temperature sensor and a temperature control mechanism, a flowcell type monitor unit including a measuring means, a fraction collector for separating samples, and a control mechanism for controlling those units.

However, although the gene analysis apparatus in accordance with the SSTEC method has such an advantage that the samples can be separated even if the difference is so small as only one base, it has such a disadvantage that complicated and time consuming operation is required, such as to dissociate the double-strand by once heating DNA after the PCR amplification, discharge the excessive primer during the cooling process, and increase in temperature of the heat block again after being cooled in order to separate the DNA which is complementary to the stationary-phase DNA probe. Moreover, much time is required for increasing in temperature of the stationaryified DNA column.

Moreover, although the above-mentioned conventional gene analysis apparatus has such an advantage that a plurality of gene analyses can be realized simultaneously from the same sample, it has such a disadvantage that a plurality of gene analyses are difficult to be realized from different kinds of samples. So, there is limit in gene analysis.

Moreover, in the conventional gene analysis apparatus, high pressure valves are inserted in the upstream and downstream positions of the columns and those valves are operatively connected to the flowcell type monitor unit so as to be selectively switched and the columns are switched in series or in parallel. Accordingly, the structure becomes complicated and the cost is increased. In addition, there is a fear that cross contamination is admixed through the valves.

On the other hand, besides the above-mentioned disadvantages, the conventional gene analysis apparatus has the following disadvantages in the respective processes.

For example, in the above-mentioned Japanese Patent Application Laid-Open No. H06-327476, a plurality of pipette tips with tip sections thereof opened, are received in a pipette tip base, the pipette tips are moved to an upper part of a container on a turn table through a pipette tip attachment section, the tips of the pipette tips are dipped for absorption in an eluent contained in the container, then the pipette tips are moved to an upper part of another container on the turn table and the eluent is discharged, and thereafter, the pipette tips are discarded. On the other hand, the container containing therein the eluent is transferred to a centrifugal separator for separation of the eluent.

However, the above-mentioned DNA extraction process has the following disadvantages. For a single extraction of DNA, a pipette tip and two containers are required and those pipette tip and containers are discarded after use. Moreover, since the eluent must be shifted into another container and finally centrifugally separated, the extracting and refining procedure of DNA is complicated and much time is required. In addition, there is a fear that cross contamination is admixed. As means for preventing the admixture of the cross contamination, for example, in Japanese Patent Application Laid-Open No. H07-289925, a plug member is attached to an intermediate part of the pipette tip, while in Japanese Patent Application Laid-Open No. H08-35971, a film is disposed at an opening part on the connection side of the pipette tip. Thus, there is such a disadvantage that the pipette tip becomes expensive to the extent of the additional costs of the plug member and the film.

Moreover, in the PCR amplifying process of the above-mentioned Japanese Patent Application No. H07-75544, two metal block type heat exchangers capable of controlling the temperature to a denaturation temperature and an annealing or extension temperature are placed side by side, and a capillary tube, which is in communication with a reaction mixture pool, is inserted into each of them. A piston, which is in association with a step motor, is slidably inserted in the capillary tube, and the reaction mixture is moved into the heat exchanger for heating through displacement of the piston. Moreover, in the above-mentioned Laid-Open Publication, a circular cylindrical heat exchanging drum is rotatably disposed about the center axis, the peripheral surface of the drum is sectioned into a plurality of segments, each segment is heated to a predetermined PCR heating temperature, a groove(s), which allows the capillary tube to be wound therearound, is formed in the peripheral surface of the drum, the drum is rotated by a predetermined angle so that a selected segment is contacted with the corresponding capillary tube to heat the reaction mixture inserted in the capillary tube, and after the heating, the drum is rotated so that another segment is contacted with the corresponding capillary tube to heat the reaction mixture sequentially.

However, in those PCR amplifiers, in the former case, the opposite sides of the capillary tube must be blocked with valves at the time of reciprocating operation of the piston. This valve causes the problem of cross contamination. Moreover, since one end of the capillary tube is in communication with the reaction mixture pool, a part of the reaction mixture, which has been heated and evaporated, flows into the reaction mixture pool to increase the concentration of the base of the reaction mixture. This makes, in some instances, it difficult to detect DNA after amplification.

In the latter case, there is such a problem that since various mechanisms are required, the structure becomes complicated and the cost is increased.

As one attempt to solve those problems, for example, Japanese Patent Application Laid-Open Publication H06-30776 makes a proposal in which a PCR heating section is disposed at the outside of a linear reaction tube for moving a reaction eluent, or otherwise, the reaction tube is wound in the form of a coil and dipped in a plurality of constant temperature bath which has been heated to the PCR heating temperature, and then, the reaction eluent is allowed to move into the tube.

However, this PCR amplifier has the following disadvantages.
Since a PCR heating section for a predetermined cycle part is required, the reaction tube becomes long. Moreover, since the entire reaction tube is wound around by 22 turns, the reaction tube becomes long and large in size and weight. Thus, a large-sized expensive pump is required for feeding the eluent, a large quantity of cleaning eluent and much time is required.

Moreover, since the detecting process of the above-mentioned Japanese Patent Application Laid-Open No. H06-327476 is carried out in accordance with the gel electrophoresis, the apparatus becomes expensive and much time is required for analysis. Moreover, separation is poor and in addition, the gel to be used must be replaced each time. Moreover, in the detecting process of the SSTC method, much time is required for increasing the temperature of the stationary-phase DNA column as previously mentioned.

It is, therefore, a main object of the present invention to provide a method for analyzing a gene and an analysis device thereof which are suited to be used, for example, as a fully automatic gene analysis device or a fully automatic gene diagnostic device which can be simplified in structure, made compact in size and light in weight, and manufactured at a low cost and in which detection of various kinds of DNA can be conducted rapidly and with precision and analysis of a plurality of genes can be made simultaneously from different kinds of samples by restraining, as much as possible, the use of a valve in an analytic system, thereby preventing admixture of cross contamination.

Another object of the present invention is to provide a method for analyzing a gene and an analysis device thereof, in which detection of various kinds of DNA can be conducted rapidly and with precision, analysis of a plurality of genes can be made simultaneously from different kinds of samples, and a double strand forming position of the DNA to be tested can be detected rapidly.

A further object of the present invention is to provide a method for analyzing a gene and an analysis device thereof, in which the stationary-phase DNA probes are increased in temperature for each analysis of the DNA to be tested, so that the stationary-phase DNA probes can be used rationally, detection of various kinds of DNA can be conducted rapidly and with precision, and analysis of a plurality of genes can be made simultaneously from different kinds of samples.

A still further object of the present invention is to provide a method for analyzing a gene and an analysis device thereof, in which the reaction eluent is heated to a predetermined temperature before the introduction into the stationary-phase DNA probes and double strands of the DNA to be tested is denatured beforehand to generate single strand, so that the time required for increasing and decreasing the temperature of the stationary-phase DNA probes is shortened and the time required for analysis is reduced remarkably.

A yet further object of the present invention is to provide a method for analyzing a gene and an analysis device thereof, in which a temperature gradient is set such that the upstream side of the stationary-phase DNA probes is equal to or higher than a denaturation temperature of the DNA and the downstream side of said stationary-phase DNA probes is equal to or lower than an extension temperature of the DNA.

### Summary of the Invention

The present invention provides a gene analysis method comprising the steps of extracting a target nucleic acid from a biological sample and amplifying a target DNA, thereafter, introducing a reaction eluent containing a predetermined DNA into a stationary-phase DNA probe having a predetermined temperature and arranged in series or in parallel and separating a DNA complementary to the stationary-phase DNA probe, characterized in that the gene analysis method comprises a plurality of stationary-phase DNA probes at least a part of which can be set to a temperature for forming a double strand of a DNA to be tested and a reaction eluent containing the same or different kinds of DNA after being amplified is introduced into the stationary-phase DNA probes.
Accordingly, detection of various kinds of DNA can be conducted rapidly and with precision, analysis of a plurality of genes can be made simultaneously from different kinds of samples, and a double strand forming position of the DNA to be tested can be detected rapidly.

Also, according to the present invention, the stationary-phase DNA probes are increased and decreased in temperature for each analysis of the DNA to be tested. Accordingly, the stationary-phase DNA probes can be used rationally, detection of various kinds of DNA can be conducted rapidly and with precision, and analysis of a plurality of genes can be made simultaneously from different kinds of samples.

Moreover, according to the present invention, the reaction eluent is heated to a predetermined temperature before introduction into the stationary-phase DNA probes and the double strand of the DNA to be tested is preliminarily denatured so that a single strand is generated. Accordingly, the time required for increasing and decreasing the temperature of the stationary-phase DNA probes is shortened and the time required for analysis is reduced remarkably.

According to the present invention, a temperature gradient is set such that the upstream side of the stationary-phase DNA probes with respect to the reaction eluent is a high temperature side and the downstream side of the stationary-phase DNA probes is a low temperature side. Accordingly, the time required for detecting DNA can be shortened and the separated complementary DNA can surely be detected.

Also, according to the present invention, a temperature gradient is set such that the upstream side of the stationary-phase DNA probes is a denaturation temperature or higher of the DNA and the downstream side of the stationary-phase DNA probes is an extension temperature or lower of the DNA. Accordingly, the double strand of a predetermined DNA can be denatured on the upstream side of the stationary-phase DNA probe.

Accordingly, there can be obviated the disadvantage involved in the conventional technique in which the double strand of the DNA is denatured beforehand, and then, the stationary-phase DNA probes are increased in temperature. And detection of the DNA can be carried out rapidly. In addition, by forming a double strand of the DNA complementary at a predetermined position of the temperature distribution, the separating position of the DNA can be detected with precision. Thus, the irregularity of the separating position can surely be detected.

Also, according to the present invention, a constant temperature zone where the complementary DNA can form a double strand is provided at an intermediate portion of the temperature distribution so that the stationary-phase DNA probes are steppingly distributed in temperature. Accordingly, the formation of the double strand of the complementary DNA can surely be detected.

Moreover, according to the present invention, a reaction eluent containing the same or different kinds of DNA is directly introduced into the stationary-phase DNA probes. Accordingly, there can be obviated the disadvantage involved in the conventional technique in which the double strand of the DNA is denatured beforehand, and then, the stationary-phase DNA probes are increased in temperature.

According to the present invention, a gene analysis device comprises a plurality of stationary-phase DNA probes at least a part of which can be set to a temperature for forming a double strand of a DNA to be tested and a reaction eluent containing the same or different kinds of DNA after being amplified can be introduced into the stationary-phase DNA probes. Accordingly, detection of various kinds of DNA can be conducted rapidly and with precision, analysis of a plurality of genes can be made simultaneously from different kinds of samples, and a double strand forming position of the DNA to be tested can be detected rapidly.

Also, according to the present invention, the stationary-phase DNA probes are increased and decreased in temperature for each analysis of the DNA to be tested. Accordingly, the stationary-phase DNA probes can be used rationally, detection of various kinds of DNA can be conducted rapidly and with precision, and analysis of a plurality of genes can be made simultaneously from different kinds of samples.

Moreover, according to the present invention, a heat block is provided to a flow passageway of the reaction eluent on the upstream side of the stationary-phase DNA probe, and the heat block can be set to temperatures at which a single strand of DNA can be formed by dissociating the double strand of DNA to be tested. Accordingly, Accordingly, the time required for increasing and decreasing the temperature of the stationary-phase DNA probes is shortened and the time required for analysis is reduced remarkably.

According to the present invention, a temperature gradient is set such that the upstream side of the stationary-phase DNA probes with respect to the reaction eluent is a high temperature side and the downstream side of the stationary-phase DNA probes is a low temperature side. Accordingly, the time required for detecting DNA can be shortened and the separated complementary DNA can surely be detected.

Also, according to the present invention, a temperature gradient is set such that the upstream side of the stationary-phase DNA probes is a denaturation temperature or higher of the DNA and the downstream side of the stationary-phase DNA probes is an extension temperature or lower of the DNA. Accordingly, the double strand of a predetermined DNA can be denatured on the upstream side of the stationary-phase DNA probe.

Accordingly, there can be obviated the disadvantage involved in the conventional technique in which the double strand of the DNA is denatured beforehand, and then, the stationary-phase DNA probes are increased in temperature. And detection of the DNA can be carried out rapidly. In addition, by forming a double strand of the DNA complementary at a predetermined position of the temperature distribution, the separating position of the DNA can be detected with precision. Thus, the irregularity of the separating position can surely be detected.

Also, according to the present invention, a constant temperature zone where the complementary DNA can form a double strand is provided at an intermediate portion of the temperature distribution so that the stationary-phase DNA probes are steppingly distributed in temperature. Accordingly, the formation of the double strand of the complementary DNA can surely be detected.

Moreover, according to the present invention, a reaction eluent containing the same or different kinds of DNA is directly introduced into the stationary-phase DNA probes. Accordingly, there can be obviated the disadvantage involved in the conventional technique in which the double strand of the DNA is denatured beforehand, and then, the stationary-phase DNA probes are increased in temperature. Thus, rapid DNA detection can be realized.

According to the present invention, a plurality of the same or different stationary-phase DNA probes are received in a plurality of columns which are arranged in parallel with each other and fluid resistance means having a larger fluid resistance than that of the column portions is disposed at each supply passageway of an eluent which is in communication with each of the columns. By reducing the flow rate of each feed passage of the eluent, the flow rate of the column part can be restrained from being varied. Thus, the parallel connection of the plural columns, the parallel treatment of the DNA detection thereof and supply of the eluent by only one feed eluent pump can be realized. In addition, the device of this type can be made compact in size and light in weight.

According to the present invention, the eluent can be supplied to each column through only one feed pump. Accordingly, the structure can be simplified, the device can be made compact in size and light in weight and the equipment cost can be reduced. In addition, by avoiding the generation of trouble caused by the pump, a smooth and stable analysis can be achieved.

According to the present invention, a multiport switch valve, into which a plurality of reaction eluents containing the same or different kinds of DNA which have been amplified, can be introduced, is disposed between the downstream side of each supply passageway of the eluent and a DNA detection apparatus, and each supply passageway of the eluent and a passageway which is in communication with each of the columns are connected to a predetermined port of the switch valve. Accordingly, genes can be analyzed simultaneously from the same or different kinds of samples, rationalization and high speed of analysis of this kind can be achieved and the cost of analysis can be reduced.

Also, according to the present invention, a tubular heat block capable of forming the temperature distribution is provided, a light source is disposed at an inner side of the heat block, the heat block is provided with transmittance means of the light source, and the stationary-phase DNA probes are arranged in such a manner as to face with the transmittance means. Accordingly, by effectively utilizing the light source, detection of the complementary gene by a plurality of stationary-phase DNA probes, for example, can be carried out.

Moreover, according to the present invention, only one or a plurality of micro-channels are formed on a base capable of forming the temperature distribution, and only one or a plurality of stationary-phase DNA probes are arranged within the channels. Accordingly, a plurality of DNA can be detected simultaneously utilizing a micropipette tip, and the device can be made compact in size and light in weight.

According to the present invention, an analysis pattern of each test sample can be displayed in a display screen of an analysis apparatus capable of detecting and analyzing a double strand forming position of the DNA to be tested by the stationary-phase DNA probes. Accordingly, the patterns of analysis of the respective test samples and variation thereof can be obtained simultaneously and easily.

The above objects, characteristics and advantages of the present invention will become more manifest from the detailed description to be described hereinafter and with reference to the accompanying drawings.

### Brief Description of the Drawings

FIG. 1 is an explanatory view showing an embodiment in which the present invention is applied to a fully-automated gene analysis employing an automatic sampler.
FIG. 2 is a sectional view showing a sampling receptacle having a bottom which is used in a nucleic acid extraction process which is applied to the present invention, showing a state in which a nucleic acid extraction reagent for decomposing a biological sample and a protein, and a nucleic acid extraction reagent for enhancing absorption of a target nucleic acid to an absorption layer are received in the sampling receptacle, with the target nucleic acid (DNA) absorbed to the absorption layer.
FIG. 3 is an enlarged sectional view taken on line A-A of FIG. 2.
FIG. 4 is a sectional view of a PCR amplifier which is applied to the present invention, showing a state in which double ironing rollers are reciprocally moved along inner surfaces between a plurality of heat blocks, thereby moving a reaction eluent in a reaction tube together with them.
FIG. 5 is an enlarged sectional view taken along B-B of FIG. 4.
FIG. 6 is a sectional view schematically showing a DNA detector which is applied to the present invention.
FIG. 7 is a sectional view showing a second embodiment of the present invention, showing a state in which a biological sample, a nucleic acid extraction reagent for decomposing a protein, and a nucleic acid extraction reagent for enhancing absorption of a target nucleic acid to an absorption layer are received in a sampling receptacle having an opening portion at a bottom portion thereof, with the target nucleic acid (DNA) absorbed to the absorption layer.
FIG. 8 is a sectional view of a PCR amplifier which is applied to a third embodiment of the present invention, showing a state in which a plurality of magnetic bodies (magnetic fluids) are reciprocally moved along inner surfaces between heat blocks, thereby moving a reaction eluent in a reaction tube together with them.
FIG. 9 is a sectional view showing, on an enlarged basis, an essential portion of a DNA detector which is applied to a fourth embodiment of the present invention, showing a state in which mutually different stationary-phase DNA probes are arranged in series in a narrow tube.
FIG. 10 is a sectional view showing, on an enlarged basis, an essential portion of a DNA detector which is applied to a fifth embodiment of the present invention and in which a plurality of narrow tubes are arranged in an axial direction of the tubes on an outer peripheral surface of a tubular heat block and a stationary-phase DNA probe is filled in each tube.
FIG. 11 is an explanatory view showing an application example of FIG. 10, in which the narrow tube is spirally wound around the outer peripheral surface of the tubular heat block.
FIG. 12 is an explanatory view showing another application example of FIG. 10, in which the narrow tube is wound around the outer peripheral surface of a tubular heat block in a direction orthogonal to an axial direction of the tube.
FIG. 13 is a perspective view showing, on an enlarged basis, an essential portion of a DNA detector which is applied to a sixth embodiment of the present invention, in which a plurality of micro-channels are formed in a micro-pipette tip and a stationary-phase DNA probe is filled in each channel.
FIG. 14 is an enlarged sectional view taken on line C-C of FIG. 13.
FIG. 15 is an explanatory view showing, partly in section, a fully automatic DNA diagnostic device which is applied to a seventh embodiment of the present invention and to an application example thereof.

### Best Mode for Carrying Out the Invention

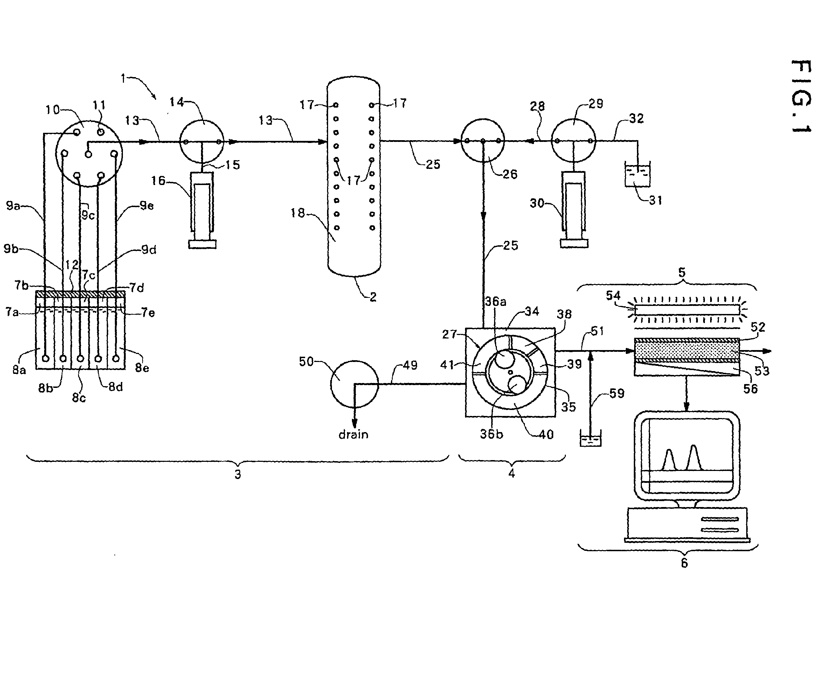

The present invention will now be described with reference to the illustrated embodiment in which the present invention is applied to a fully automatic gene analysis device capable of automatically carrying out a series of processes ranging from extraction of DNA, RNA or the like, extraction of DNA in this embodiment to amplification of DNA and detection/analysis of DNA. In FIGS. 1 through 6, reference numeral 1 denotes a gene analysis device. This gene analysis device 1 comprises a nucleic acid extraction device 3 including an automatic sampler 2, a DNA amplification device 4, a DNA detection device 5 and a control/analysis device 6.

The nucleic acid extraction device 3 includes a plurality of nucleic acid extraction reagent storage receptacles 7a through 7e, in which prescribed nucleic acid extraction reagents 8a through 8e such as, for example, protease, chaotropic agent, phenol or chloroform, 70 % ethanol, a mixed eluent of eluent, DNA synthetic material and primer, distilled water and the like.

Lower end portions of reagent conduits 9a through 9e are laid in the storage receptacles 7a through 7e, respectively.
Upper end portions of the conduits 9a through 9e are connected to all ports of a first switch valve 10 only excepting an air port 11. In FIG. 1, reference numeral 12 denotes a lid plate for closing the opening portions of the storage receptacles 7a through 7c. The lid plate 12 is provided with insertion holes (not shown) for the reagent conduit tubes 9a through 9e. The first switch valve 10 includes six ports. An injection tube 13, which can communicate with each of the six ports, is connected to the automatic sampler 2.

In FIG. 1, reference numeral 14 denotes a three-way valve which is inserted in the injection tube 13. A passageway 15, which can communicate with a port of the valve 14, is provided with a syringe pump 16 serving as feed eluent means. Operation timing and operation displacement of the syringe pump 16 are controlled by a computer installed in the control/analysis device 6, which computer stores therein a gene analysis system and a nucleic acid extraction system according to the present invention, thereby enabling to sequentially inject the nucleic acid extraction reagents 8a through 8e into sampling receptacles 17.

The automatic sampler 2 has a sampling function and an injection function. The automatic sampler 2 is provided at a front portion thereof with a sampling rack 18 which can receive therein the sampling receptacles 17. A plurality of through holes, into which the sampling receptacles 17 can be inserted, are formed in the rack 18.

A heat block 20 is disposed immediately under the sampling rack 18. The heat block 20 can be controllably heated to a predetermined temperature, 58 degrees C in this embodiment. A plurality of recesses 21 capable of receiving therein the lower end parts of the sampling receptacles 17, are formed in an upper surface of the block 20.

Each sampling receptacle 17 is formed in a bottomed cylindrical configuration from a synthetic resin such as polypropylene. A generally lower half part of the sampling receptacle 17 is tapered 17a. A strip-shaped absorption layer 22 having a small thickness is formed at a predetermined position of an inner surface of the receptacle 17.

The surface of the absorption layer 22 is formed in a rough surface capable of absorbing a predetermined DNA, i.e., template DNA. In this embodiment, pulverized glass beads or silica gel beads are thermofused in a belt shape on an inner surface of the receptacle 17 in the circumferential direction.

A lower end part of the absorption layer 22 is located above an eluent surface of a blood (so-called whole blood including blood plasma and red blood cells) 23 as a biological sample received in the sampling receptacle 17 and above a surface of a mixed eluent of protease as the nucleic acid extraction reagent 8a which is capable of decomposing protein dropped onto the blood 23.

The layer thickness of the absorption layer 22 is set such that when a chaotropic agent 8b as a nucleic acid extraction reagent 8b for enhancing the absorption of a target nucleic acid to the absorption layer 22 is dropped onto the mixed eluent, the surface of the mixed eluent 8a, 8b exceeds at least the lower end part of the absorption layer 22. In this case, the surface of the mixed eluent 8a, 8b may exceed the upper end part of the absorption layer 22.

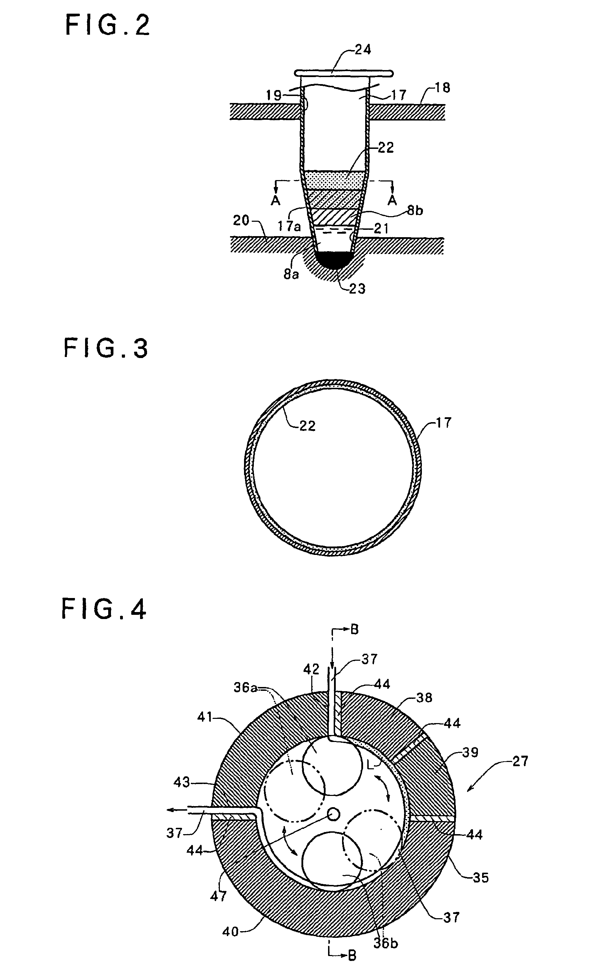

In FIG. 2, reference numeral 24 denotes a cap which is removably attached to an upper end part of the sampling receptacle 17.

A container (not shown) receiving therein a synthetic agent including DNA polymerase, primer DNA and dNTP is disposed at the sampling rack 18. A required amount of the synthetic agent is injected into the sampling receptacle 17 in which the template DNA is extracted, and the reaction eluent L is transferred to a PCR amplifier 27 through a feed eluent tube 25 and a second switch valve 26.

The second switch valve 26 is constituted of a three-way valve. A conduit 28 is connected to a predetermined position of the three-way valve 26, and a syringe pump 30 as a feed means is connected to the conduit 28 through a three-way valve 29.

Operation timing and operation displacement of the syringe pump 30 are controlled by the computer of the control/analysis device 6, which computer stores therein a gene analysis system and a nucleic acid extraction system according to the present invention, thereby enabling to transfer the reaction eluent to the PCR amplifier 27 and enabling to supply a cleaning eluent 31 to the PCR amplifier 27 through a feed eluent tube 32 after the PCR amplification.

The PCR amplifier 27 is installed at the inside of a housing 34. The PCR amplifier 27 includes an aluminum alloy-made hollow cylindrical cylinder block 35, and a plurality of ironing rollers 36a, 37b as movers which are received in the hollow cylindrical cylinder block 35 in such a manner as to be able to roll and reciprocally move along the inner surface of the block 35. As the ironing rollers 36a, 36b, peristaltic rollers which constitute a peristaltic pump, are used in this embodiment.

The cylinder block 35 has a soft elastic tube 37 circularly turned around an inner surface thereof as a reaction tube. The cylinder block 35 has a height in the axial direction which height is equal to the turning width of the elastic tube 37. In this embodiment, the elastic tube 37 is circularly turned by about 270 degrees which is equal to less than one turn.

The cylinder block 35 is constituted of three heat blocks 38 through 40 each of which has a sector-shaped configuration in section, and a connecting block 41 having a sector-shaped configuration in section. This connecting block 41 is disposed between the heat block 38 and the heat block 40.

In this embodiment, 8 elastic tubes 37 for allowing the reaction eluent L, which contains DNA of the same or different kinds of test samples, to move therein, are turned around the inner surface of the cylinder block 35 such that an axial height equal to the turning width is formed.

Accordingly, 8 feed eluent tubes 25 are introduced to the PCR amplifier 27, and the corresponding number of second switch valves 26 and conduits 28 are arranged on the syringe pump 30.

The heat blocks 38 through 40 are capable of controllably heating the PCR amplifier to a predetermined temperature which can be realized. Of all the heat blocks, the heat block 38 is heat to approximately 95 degrees C, so that the double strand of DNA in the reaction eluent L within the elastic tube 37 is denatured to generate a single strand.

The heat block 39 is heated to approximately 60 degrees C and it undertakes the annealing process for connecting two kinds of primers to the single strand DNA for annealing.

The heat block 40 is heated to approximately 72 degrees C and it undertakes an extension process for synthesizing a double strand DNA through a DNA polymerase and copying a complementary DNA.

Those heat blocks 38 through 40 are determined in length of heating parts, i.e., inner peripheral surfaces thereof in accordance with the heating time with respect to the reaction eluent L and arranged in the order of heating.

The connecting block 41 is removably attached to the adjacent heat blocks 38, 40 or the housing 34 by machine screws or the like. Inlet and outlet grooves 42, 43 of the elastic tube 37 are formed on opposite end parts of the connecting block 41. Owing to this arrangement, when the connecting block 41 is removed, the elastic tube 37 can be inserted into and withdrawn from the grooves 42, 43. Accordingly, no elastic tube 37 is arranged on the inner surface of the connecting block 41.

Therefore, it is preferred that the inner surface of the connecting block 41 is formed large in thickness and smooth to the ironing extent of the elastic tube 37. In the illustration, reference numeral 44 denotes a heat insulating material disposed between the heat blocks 38 through 40 and the connecting block 41.

The elastic tube 37 is constituted, for example, of a silicon tube having a soft heat resisting property. One end of the elastic tube 37 is connected to downstream side of the feed eluent tube 25 and the other end is connected to a transfer tube as later described.

The elastic tube 37, as shown in FIG. 4, is introduced into the cylinder block 37 through the inlet groove 42, laid on the same plane along the inner surfaces of the heat blocks 38, 39 and 40, and pulled out to the outside of the cylinder block 37 through the outlet groove 43.

Accordingly, the elastic tube 37 is arranged within the cylinder block 37 by approximately 270 degrees, i.e., equal to or less than one turn, only excluding the connecting bock 41.

Thus, the elastic tube 37 can be made compact in size. Therefore, even if 8 elastic tubes 37 should be used as mentioned above, the turning width can be only eight times the diameter of the elastic tube 37. Thus, the cylinder block 37 can be made compact in size and light in weight.

The ironing rollers 36a, 36b are each formed in a circular cylindrical configuration having a length generally equal to the height of the cylinder block 37. The ironing rollers 36a, 26b are arranged in point symmetry at a predetermined space. Opposite ends of the ironing rollers 36a, 36b are rotatably supported by pins 46 projecting from a connecting rod 45. The connecting rods 45, 45 are connected to a rotational shaft 47 and associated with a motor 48 which is capable of rotating the shaft 47 normally and reversely.

At the time of PCR amplification process carried out by the heat blocks 38 through 40, i.e., at the time of normal rotation of the ironing rollders 36a, 36b, the motor 48 is stopped for each heating in the respective processes. After the passage of a predetermined heating time, the motor 48 is driven again to carry out the amplifying operation in the respective processes.

After one cycle of the PCR amplification process is finished, the ironing rollers 36a, 36b are reversely rotated to return to their original positions with the stopping time inserted therebetween. Then, the PCR amplification process is resumed. This reciprocal angular motion can be executed by the cycle portion, 30 cycles in this embodiment, required for the PCR amplification.

The reciprocal motion angle of the ironing rollers 36a, 36b is, as shown in FIG 4, set to be approximately 90 degrees. The ironing rollers 36a, 36b are turned normally and reversely along the inner surface of the cylinder block by the above-mentioned angle portion. During this time, opposite end parts of the elastic tube 37 are closed air tight so that the inside reaction eluent L and water vapor are prevented from leaking out. In the illustration, reference numeral 49 denotes an eluent discharge tube which is disposed at the PCR amplifier 27. A stop valve 50 is inserted in the eluent discharge tube 49.

A transfer tube 51 is connected to the downstream side of the elastic tubes 37. The other end of the transfer tube 51 is introduced to the detector 5, which is an UV detector in this embodiment. A column 52, which is a narrow tube, is removably attached to the transfer tube 51 in the detector 5.

Accordingly, in this embodiment, 8 transfer tubes 51 are introduced into the detector 5, and a predetermined column 52 is attached to the transfer tubes 51. The column 52 is constituted of a transparent glass tube having a small diameter, and a predetermined stationary-phase DNA probe 53 filled therein, or a predetermined stationary-phase probe 53 coated on an inner wall of the glass tube.

The column 52 is attached to the transfer tube 51, so that the reaction eluent containing the PCR amplified DNA to be tested can directly be introduced therein. In the illustration, reference numeral 54 denotes an UV lamp which is disposed immediately above the column 52, and reference numeral 55 denotes an optical filter which is disposed between the column 52 and the UV lamp 54.

A heat block 56 having a length generally equal to that of the column 52 is disposed at a location proximate to the column 52, at a located immediately under the column 52 in this embodiment. A high temperature block 57 and a high temperature block 57 having Peltier effect are disposed at opposite sides of the heat block 56. Due to the heating and heat transmitting actions, a constant temperature distribution is formed along the longitudinal direction of the heat block 56. In this case, a proper cooling means such as a fan may be disposed at the low temperature block 58 side in accordance with necessity.

The above-mentioned temperature distribution forms, as shown, a temperature gradient which is gradually decreased in the moving direction of the DNA to be tested and which includes a double strand forming temperature of the DNA to be tested. In this embodiment, the temperature distribution is set such that the high temperature block 57 side is 85 degrees C which is higher than the double strand forming temperature of the DNA to be tested and the low temperature block 58 side is 30 degrees C which is lower than the double strand forming temperature of the DNA to be tested. Owing to this arrangement, the DNA to be tested can surely be detected.

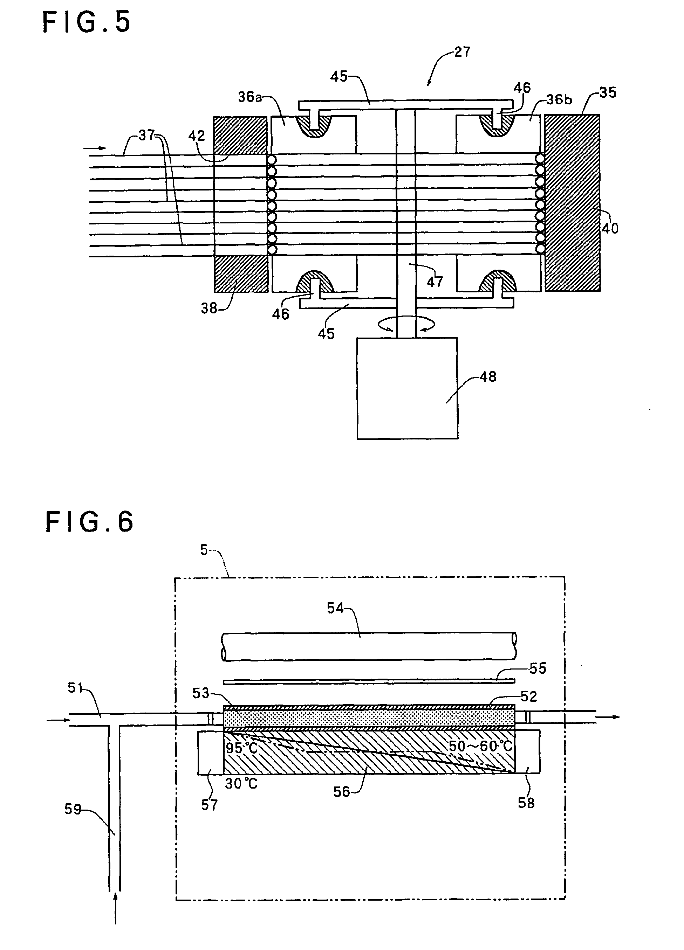

In this case, it is also accepted that a heat block (not shown) capable of heating to a predetermined temperature is attached to an intermediate position of the heat bock 56, and a temperature region of 50 degrees C through 60 degrees C is disposed at an intermediate part in the length direction of the heat block 66 so that a temperature distribution is formed which stepwise varies in three steps of 95 degrees C, 50 degrees C through 60 degrees C and 30 degrees C as indicated by the chain line of FIG. 6.

In this way, by forming stepwise the temperature distribution of the heat block 56 and providing a large temperature region of 50 degrees C through 60 degrees C suitable for forming a double strand of the DNA to be tested at its intermediate part, the nucleic acid to be tested can surely be detected and reliability on detection can be enhanced.

In the illustration, reference numeral 59 denotes an inlet tube of an intercurrent dye as a fluorescent eluent which is disposed at the upstream side of the detector 5. After the separation of the DNA to be tested, i.e., after the formation of a double strand, the intercurrent dye is introduced into the column 52 so that the double strand forming position of the DNA to be tested can be fluorescently displayed.

After the introduction into the intercurrent dye, the double strand forming position of the DNA to be tested is fluorescently detected by irradiation of the UV lamp 54 and scanning the column 52. The detection signal is inputted into the computer of the control/analysis device 6 in order to calculate and analyze the presence/absence of the target DNA which is complementary to the stationary-phase DNA probe 53, the peak position and the peak value.

The gene analysis device thus constructed roughly comprises a nucleic acid extraction device 3, a PCR amplifier and the DNA detector 5. Of those components, the sampling rack 18 of the nucleic acid extraction device 3 has an absorption layer 22 formed on the inner surface. Use of only one sampling rack 18 is good enough to extract a predetermined DNA or RNA unlike in the conventional technique in which 4 to 6 sampling racks are required for one sampling operation.

The sampling rack 18 may be made as follows. Glass beads or silica gel beads in the form of powder are thermofused in a strip shape to a predetermined position of an inner surface of a conventional bottomed container which is made of synthetic resin so that a film-like or strip-like absorption layer 22 is formed. By doing so, there can easily be made a sampling rack which is simple in structure, without a need of a special equipment and at a low cost.

In the DNA amplifier 4, the connecting block 41 is removably attached to the heat blocks 38 through 40. After removing the block 41, the elastic tube 37 is laid on the inner surface of the cylinder block 35.

At that time, one elastic tube 37, which is necessary for amplifying the target DNA is turned around the inner surface of the cylinder block 35 by a basic cycle portion, approximately 270 degrees in this embodiment.

Accordingly, since it is no more required, unlike in the conventional technique, to turn or wind the elastic tube 37 by plural cycle portions, the elastic tube 37 can be miniaturized, the cylinder block 35 and the heat blocks 38 through 40 can be made compact in size and light in weight, and the installation space can be made compact.

Moreover, the PCR amplifier 4 is provided with a plurality of ironing rollers 36a, 36b which can reciprocally move by a constant angle. Those ironing rollers 36a, 36b close air tight the opposite ends of the reaction eluent L and move the reaction eluent L in that condition.

Accordingly, it is no more required, unlike in the conventional technique, to close the opposite end parts of the elastic tube with valves. Thus, the number of parts can be reduced to that extent. This makes it possible to simplify the structure of the device. Moreover, the cross contamination can be prevented from admixing due to insertion of the valves and the reaction eluent L can be prevented from being evaporated by heating.

On the other hand, in the DNA detector 5, since the heat block 56 capable of forming a temperature gradient in the longitudinal direction is disposed at a location proximate to the column 52 in which the stationary-phase DNA probe 53 is filled or coating is applied to the inner wall, the double strand forming temperature of the DNA to be tested can be obtained rapidly compared with the conventional technique in which the heat block is increased in temperature at a predetermined temperature gradient.

Moreover, since the heat block 56 forms a temperature gradient in the longitudinal direction over the above and under the temperature of the nucleic acid to be tested, the DNA to be tested can surely be detected.

In case a target DNA is analyzed by the gene analysis device thus constructed, a target nucleic acid that is a template DNA in this embodiment, is extracted to the sampling receptacles 17 first.

That is, in the sample rack 18 of the automatic sampler 2, 8 sample receptacles 17 used for one time DNA extraction, for example, are prepared and a predetermined quantity of the same or different types of blood 23 as a biological sample is injected therein. In that case, the eluent surface of the blood 23 is located under the lower end part of the absorption layer 22.
This state is as shown in FIG. 2.

Almost simultaneously, various kinds of nucleic acid extracting chemicals 8a through 8e such as, for example, protease, chaotropic agent, phenol or chloroform, 70% ethanol, distilled water and the like, are received in the nucleic acid extracting chemical storage receptacles 7a through 7e.

For example, protease as the nucleic acid extracting chemical 8a capable of decomposing protein is injected in the sampling receptacles 17 through the syringe pump 16 in such a manner as to be non-contacted with the absorption layer 22 and then heated to 58 degrees C by the heat block 20 to decompose the protein contained in the blood 23. And the blood cell component, which does not contribute to extraction of DNA, is dissolved.

In this case, the eluent surface after the blood 23 and the protease 8a are admixed is located under the lower end part of the absorption layer 22 as shown in FIG. 2.

Then, for example, a chaotropic agent as the nucleic acid extraction reagent 8b for enhancing absorption of a target nucleic acid to the absorption layer 22 is injected in the respective sampling receptacles 17 through the syringe pump 16, so that the target DNA contained in the blood 23 is enhanced in absorption to the absorption layer 22.

In this case, the eluent surface of the mixture of the blood 23, the protease 8a and the chaotropic agent 8b is, as shown in FIG. 2, located above the lower end part of the absorption layer 22 and the target DNA is absorbed to the absorption layer 22.

In this way, at the time of injection of the protease 8a, contact between the target DNA and the absorption layer 22 is avoided and at the time of injection of the chaotropic agent, the target DNA is absorbed to the absorption layer 22 so that the sampling efficiency of the DNA is enhanced.

That is, when the dissolved blood cell component is contacted with the absorption layer 22 at the time of injection of the protease 8a, an enzyme for decomposing DNA is discharged to decompose the DNA which is to be analyzed.

When the chaotropic agent is injected in that state, only about 50% of DNA can be sampled. Therefore, the absorption layer 22 is not formed on the bottom part of the sampling receptacle 17. Instead, the absorption layer 22 is formed at the predetermined location spaced apart from the bottom part.

Thereafter, for example, phenol and/or chloroform as the nucleic acid extraction reagent 8c is injected in the respective sampling receptacles 17 through the syringe pump 16, so that an undesired substance such as the dissolved blood cell component is cleaned and discharged to the outside of the system.

Moreover, 70% ethanol of the nucleic acid extraction reagent 8d is injected in the respective sampling receptacles 17 and the remaining eluent such as a cleaning eluent in the receptacles 17 is discharged to the outside of the system.

When the respective sampling receptacles 17 have been washed and cleaned in the manner as just mentioned above, the distilled water 8d as an eluent is injected in the receptacles 17 through the syringe pump 16, so that DNA is separated from the absorption layer 22 and the target DNA is extracted.

Then, a synthetic agent including DNA polymerase, primer DNA and dNTP is injected in the receptacles 17, and the respective reaction eluent L is transferred to the PCR amplifier 27 through the feed eluent tube 25 and the second switch valve 26. In the PCR amplifier 27, the ironing rollers 36a, 36c is located slightly counterclockwise from the position indicated by a stationary line of FIG. 4 at the time of introduction into the reaction eluent L and the inlet part of the elastic tube 37 is opened.

Accordingly, the reaction eluent L is moved to the ironing roller 36b side through the elastic tube 37. When a desired quantity of the reaction eluent L has been filled in the tube 37, this state is detected by a sensor (not shown) such as a photo coupler device and the detection signal is inputted in the motor 48 so that the motor 48 is rotated normally and reversely.

Thus, while rotating, the ironing rollers 36a, 36b are turned clockwise in FIG. 4 within the cylinder block 35, and the ironing rollers 36a, 36b crush the opposite end parts of the elastic tube 37 and iron the reaction eluent L, and then, they are moved in the order of the heat blocks 38 through 40 for heating.

The heat blocks 38 through 40 are preliminarily heated to the predetermined PCR amplification temperatures 95 degrees C, 60 degrees C and 72 degrees C, respectively. Whenever the reaction eluent L is moved along the heat blocks 38 through 40, the target DNA is thermally denaturated, annealed, elongated and amplified.

At that time, the motor 48 stops rotation whenever the ironing rollers 36a, 36b are moved to the boundary part between the adjacent heat blocks. After the passage of a predetermined time, the motor 48 is driven again for rotation to allow the respective heat blocks 38 through 40 to heat for a predetermined time, i.e., to execute the respective PCR amplification processes for a predetermined time, thereby obtaining the amplification action.

When the ironing rollers 36a, 36b have been moved in the direction as indicated by a chain line of FIG. 4 and the basic cycle of the PCR process has been finished, the motor 48 stops rotation. Then, after the passage of a predetermined time, the motor 48 is rotated reversely.

Therefore, while rotating, the ironing rollers 36a, 36b are turned counterclockwise in FIG. 4 at a stretch within the cylinder block 35. At that time, the ironing rollers 36a, 36b crush the opposite end parts of the elastic tube 37, iron the inside reaction eluent L and moves to the original position for the initial reciprocal motion.

Thereafter, the motor 48 is rotated normally. While rotating, the ironing rollers 36a, 36b are once again reciprocally moved and turned clockwise in FIG. 4 within the cylinder block 35, iron the inside reaction eluent L and intermittently move in the order of the heat blocks 38 through 40 for heating.

Thereafter, the motor 48 is intermittently rotated normally to execute the PCR amplification processes one after another. After the amplification process for one cycle portion is finished, the motor 48 is rotated reversely to return the ironing rollers 36a, 36b to their original positions.

Such reciprocal angular motion is repeated and the amplification process is executed by a predetermined cycle portion which is 30 cycles in this embodiment. Thereafter, the PCR amplification is finished.

In this way, in the PCR amplification, the opposite end parts of the elastic tube 37 are closed with the ironing rollers 36a, 36b and moved with the reaction eluent enclosed therein air tight. Accordingly, the opposite end parts of the elastic tube are not required to close and therefore, the number of parts can be reduced to that extent and the structure can be simplified.

Moreover, admixture of cross contamination caused by insertion of the valves and leakage of water vapor caused by heating of the reaction eluent L can be prevented, and the reliability of analysis can be enhanced. In addition, since the ironing rollers 36a, 36b are not in contact with the reaction eluent L, the problem of admixture of cross contamination caused by the rollers 36a, 36b is not arisen.

After the above-mentioned PCR amplification, the reaction eluent L containing the amplified DNA, contains the DNA which is in a double strand state, and excessive primer. Such reaction eluent L is moved from the respective elastic tubes 37 to the detector 5 via the transfer tube 51.

The column 52 is attached to the detector 5, and the reaction eluent L is moved through the stationary-phase DNA probe within the column 52.

The stationary-phase DNA probe 53 is heated to a predetermined temperature gradient through the heat block 56 which is disposed proximate to the column 52. This temperature gradient forms a temperature distribution which is gradually reduced in the longitudinal direction of the column 52, i.e., in the moving direction of the reaction eluent L.

That is, the opposite end parts of the heat block 56 are heated or cooled to 95 degrees C and 30 degrees C through the high temperature block 57 and the low temperature block 58 which are disposed at the opposite end parts of the heat block 56. The intermediate part of the heat block 56 forms a temperature gradient which is gradually reduced in the longitudinal direction by the heat conduction. The stationary-phase DNA probe 53 within the column 52 also forms a similar temperature distribution.

Accordingly, the double strand forming temperature of the DNA to be tested can more rapidly be obtained than the conventional technique in which the heat block is increased in temperature at a predetermined temperature gradient. Thus, the DNA to be tested can surely be detected. Moreover, the reaction eluent L containing the amplified DNA is transferred directly to the column 52 to separate the target DNA.

Accordingly, it is no more required to have such a complicated and time consuming process as to dissociate the double-strand by once heating DNA after the PCR amplification, discharge the excessive primer during the cooling process, and increase in temperature of the heat block again after being cooled in order to separate the target DNA.

When the amplified reaction eluent L is moved through the stationary-phase DNA probe 53 under the above-mentioned condition, first, the DNA which is complementary to the stationary-phase DNA probe 53 on the high temperature side is denatured in double strand and gradually cooled after denaturation and shifted to the annealing process where the DNA to be tested begins to form the double strand in the temperature region of 50 degrees C through 60 degrees C.

After the DNA to be tested begins to form the double strand, the intercurrent dye is delivered to the column 52 through the inlet tube 52 to fluorescently label the double strand forming position so that this position is scanned under irradiation of the UV lamp 54 and fluorescently detected.

Accordingly, the double strand forming position of the DNA to be tested and its fluorescent intensity are correctly detected, and the detecting signal is inputted into the control/analysis device 6. The excessive primer, etc. contained in the reaction eluent L are allowed to pass the stationary-phase DNA probe 53 as they are and discharged to the outside of the column 52.

The detecting signal is inputted into the computer of the control/analysis device 6 in which presence/absence of the target nucleic acid, the separating position and the peak value are calculated and analyzed.

After the series of DNA are analyzed, the cleaning eluent 31 is sent to the PCR amplifier 27 through the syringe pump 30 so that the elastic tube 37 is cleaned. In this case, since the elastic tube 37 is equal to single turn or less and small, a little quantity of cleaning eluent is good enough.

The heat block 56 is heated, the entire area of the column 52 and stationary-phase DNA probe 53 is heated to 95 degrees C, and the flow buffer is flowed for cleaning. By doing so, the probe 53 can be used repeatedly.

A heat block, which is entirely heated to 85 degrees C, is provided separately in order to heat more quickly. By doing so, the cleaning time can be reduced.

In the above-mentioned PCR amplifier 27, the elastic tube 37 is turned around the inner surface of the cylinder block 35 by about 270 degrees. The elastic tube 37 may be turned around by one turn or more. By doing so, the number of amplification cycle can be reduced and the amplifying time can be reduced.

It is also accepted that the elastic tube 37 is disposed linearly instead of the ring shape, the heat blocks 38 through 40 for single or plural cycle portion are disposed at the outer side of the elastic tube 37, and a plurality of ironing rollers 36a, 36b are disposed at the other side of the outer side of the tube 37, so that by linearly reciprocally moving the rollers 36a, 36b, the DNA contained in the reaction eluent L within the tube 37 is heated for amplification.

By doing so, the heat blocks 38 through 40 can be simplified in structure and the PCR amplifier 27 can be manufactured easily and at a low cost to that extent.

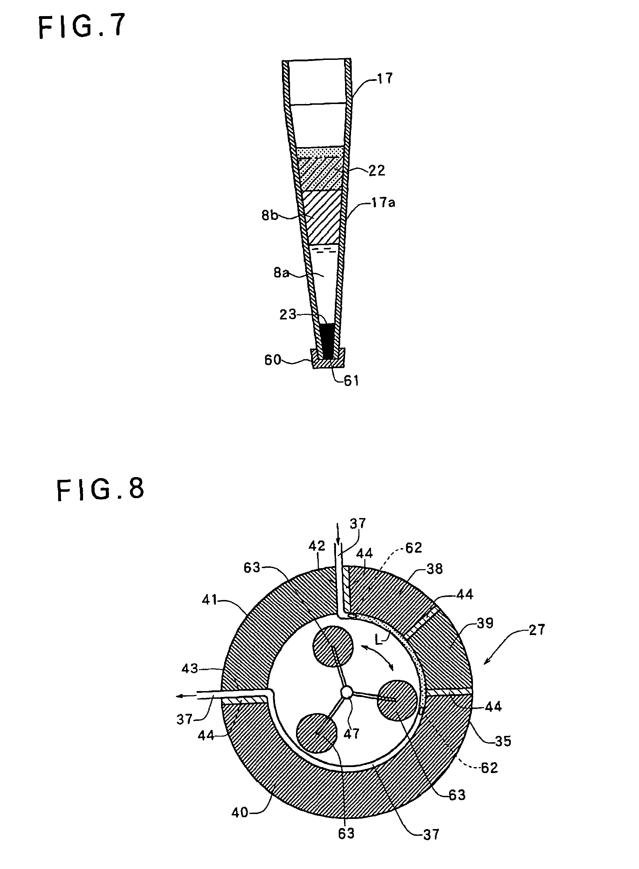

FIGS. 7 through 15 show other embodiments of the present invention, in which those component parts corresponding to the above-mentioned embodiment are denoted by same reference numeral.

Of those Figures, FIG. 7 shows the second embodiment of the present invention. This second embodiment employs a sampling tip which is a modification of the sampling receptacle 17. This tip 17 is formed in a tapered tubular shape. A cap 61 is removably attached to an opening part 60 on the tapered side, and an absorption layer 22 is formed on the inner surface of a predetermined position of the tip 17 in the same manner as mentioned above.

At the time of DNA extraction, the cap 61 is attached to the opening part 60, the tip 17 is erected with the opening part 60 facing downward, and the blood 23 is received in the tip 17 first. Then, protease 8a and chaotropic agent 8b are injected in the tip 17 in the order. At the time of injection of the chaotropic agent, the eluent surface of the chaotropic agent is positioned at a lower end part or higher of the absorption layer 22 and the target DNA is absorbed to the absorption layer 22.

After absorption of the DNA to the absorption layer 22, the cap 61 is removed to discharge the protease 8a and the chaotropic agent 8b through the opening part 60. Thereafter, various kinds of cleaning reagents are injected into the tip 17 from the top, admixed and then, discharged from the opening part 60. In other words, the reagents are kept in a normally discharging state in order to eliminate the trouble for injecting and discharging the reagents from the top for each procedure.

After cleaning, the cap 61 is attached to the opening part 60 and a distilled water is injected in the tip 17 so that the target DNA is separated from the absorption layer 22, a synthetic eluent such as DNA polymerase, primer and dNT is injected therein so that they are transferred to the PCR amplifier 27. The amplification process and the detection process to follow are conducted in the same manner as previously mentioned.

FIG. 8 shows the third embodiment of the present invention. In this third embodiment, a modification of the PCR amplifier 27 is employed. A plurality of magnetic members 62 such as a magnetic fluid as a mover are spacedly arranged within the elastic tube 37. A reaction eluent L containing a template DNA is introduced between the adjacent magnetic members 62.

On the other hand, a plurality of circular cylindrical magnets 63, which are capable of absorbing the magnetic member 62, are arranged at equal angular position within the cylinder block 35. Those magnets 63 are connected to the rotational shaft 47 through a connecting rod 47, and the rotational shaft 47 is associated with the motor 48.

Then, the magnets 63 are reciprocally angularly rotated and the magnetic member 62 is also rotated together with the magnets 63, so that the reaction eluent L received between the adjacent magnetic members 62, 62 is moved along the inner surfaces of the heat blocks 38 through 40 and the reaction eluent L is heated to amplify a target DNA.

In this PCR amplifier 27, the magnets 63 are non-contacted or lightly contacted with the elastic tube 37 so that the elastic tube 37 is prevented from being worn or damaged.

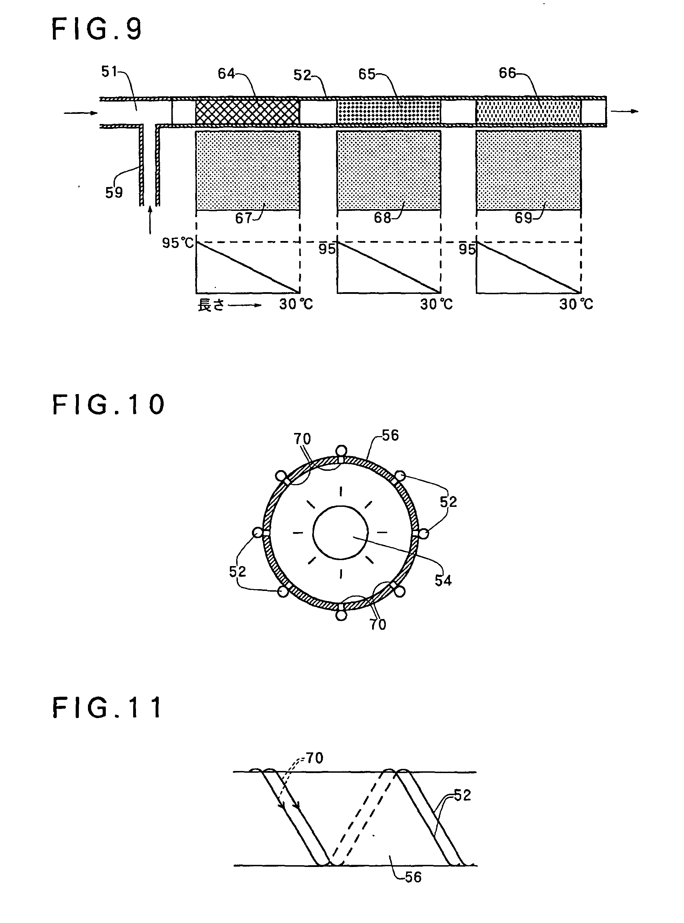

FIG. 9 shows the fourth embodiment of the present invention. In this fourth embodiment, a modification of the detector 5 is employed. In this modification, a plurality of different stationary-phase DNA probes 64 through 66 are mutually spacedly linearly filled or a plurality of different stationary-phase DNA probes 64 through 66 are linearly coated on the inner wall of the column 52. Heat blocks 67 through 69 having a generally same length as the DNA probes 64 through 66 are disposed proximate to the outer side of the column 52.

Those heat blocks 67 through 69 are each provided at opposite end parts thereof with a high temperature block 57 and a low temperature block 58 (not shown), so that the heat blocks 67 through 69 form a temperature distribution which varies in temperature along the longitudinal direction of the heat blocks 67 through 69 or which varies stepwise in temperature in the same manner as in the above-mentioned embodiment. Owing to this arrangement, a plurality of DNA contained in the reaction eluent L moving through the column 52 can be detected at a time. The temperature distribution is the same as in the above-mentioned embodiment.

FIGS. 10 through 12 show the fifth embodiment of the present invention. In this fifth embodiment, a heat block 56 formed of an aluminum tube capable of forming a temperature gradient in the direction of the axis of the tube is disposed at the outer side of a mercury lamp as a light source 54, a plurality of slits 70 as transmitting means are formed in the peripheral surface of the block 56 along the direction of the axis of the tube, and a plurality of columns 52 are arranged at the outer side of the slits 70, so that the light coming from the light source 54 is irradiated to the columns 52 through the slits 70. That is, in this embodiment, by effectively utilizing the light source 54, a plurality of nucleic acids can be detected.

FIGS. 11 is an application example of FIG. 10. In this application example, a single or plurality of slits 70 are spirally formed in the outer peripheral surface of the heat block 56, and the column 52 is wound along the slits 70 to elongate the column 52. By doing so, the detection precision of DNA is enhanced.

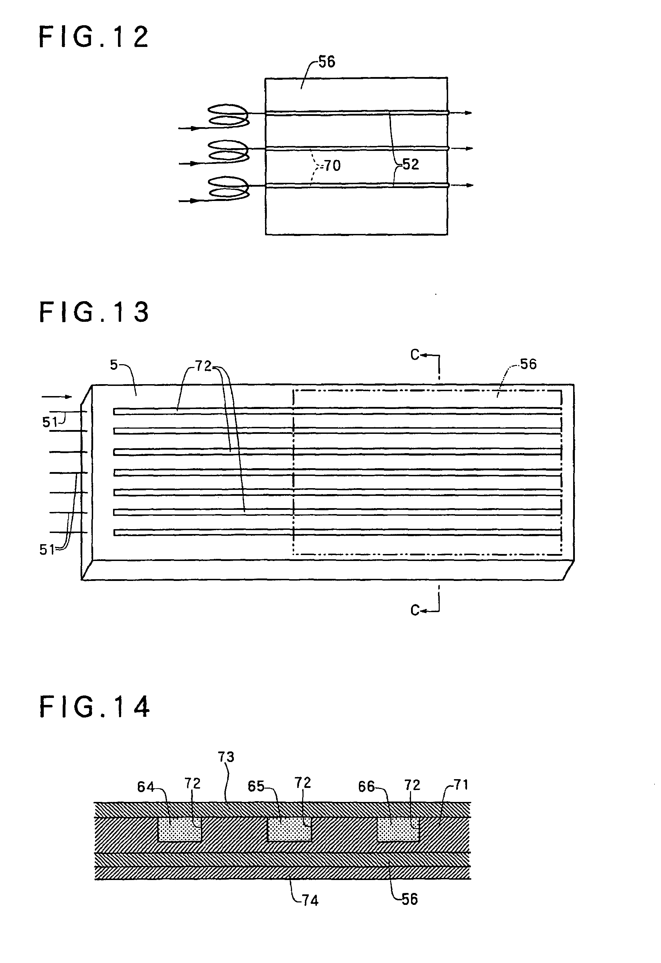

FIG 12 is another application example of FIG. 10. In this application example, a plurality of slits 70 are wound around in a direction intersecting the direction of axis of the heat block 56, in a direction orthogonal to the axis in this example, instead of in a spiral pattern as mentioned above, so that the heat block 56 can be shortened, compact in size and light in weight and the column 52 can be elongated. By doing so, the detection precision of DNA is enhanced.

FIGS. 13 and 14 show the sixth embodiment of the present invention. In this sixth embodiment, the detector 5 is constituted of a micropipette tip so that the microchemical analysis can be realized.

That is, the detector 5 for the microchemical analysis is formed in a thin plate-like configuration having, for example, a vertical length of 25 mm, a horizontal length of 50 mm and a thickness of 2 mm. In this detector 5, the surface of a base 71 such as a silicon wafer is subjected to etching treatment so that a plurality of microchannels 72 (fine flow passage) are formed. In this embodiment, the microchannels 72 are each formed in a configuration having a width of 50 µ m. a depth of 20 µ m and a pitch of 125 µm.

A cover 73, which is made of silica glass, is thermofused onto the base 71 to close the opening parts of the microchannels 72, and the same or different stationary-phase DNA probes 64 through 66 are filled in the microchannels 72, or the same or different stationary-phase DNA probes 64 through 66 are coated on to the inner walls of the microchannels 72, or a plurality of different stationary-phase DNA probes 64 through 66 are spacedly filled in the microchannels 72 in the longitudinal direction.

A heat block 56 having an aluminum film-like configuration is welded to the undersurface of the base 71, and a bottom plate 74 such as silicon wafer is thermofused to the undersurface of the block 56.

A temperature gradient is formed on the heat block 56 in which the heating temperature is gradually reduced along the flowing direction of the reaction eluent, so that the DNA, which is complementary to the stationary-phase DNA probes 64 through 66, can be separated. In this way, a plurality of DNA can be detected at a time by the detector 5 constituted of a micropipette tip in this embodiment.

In the above embodiment, DNA is extracted by the nucleic acid detector 3. Even in case of extraction of RNA, the substantially same operation can be made. The extracted RNA is reversely transferred so that it is converted into DNA, and then the synthetic agent containing DNA polymerase is added thereto to make a reaction eluent L. The reaction eluent L thus made is then transferred to the PCR amplifier 27.

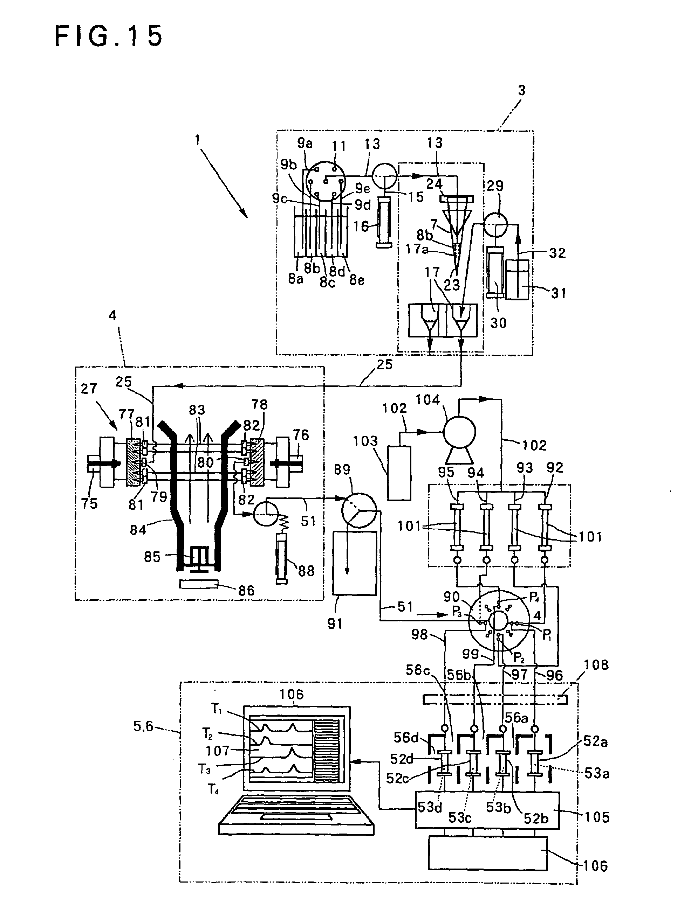

FIG. 15 shows the seventh embodiment of the present invention. In this seventh embodiment, a desired quantity of reaction eluent L composed of the same or different kinds of samples generated by the automatic sampler 2 are mutually spacedly delivered to a single feed eluent tube 25 in a sequential manner. This reaction eluent L is introduced into the DNA amplifier 4.

In the DNA amplifier 4, the ironing rollers 36a, 36b, the motor 48 and the heat blocks 38, 39, 40 are omitted. Instead, a pair of turnable disc-like valve heads 77, 78 are arranged in opposing relation through motors 75, 76.

The motors 76, 77 are controlled in operation by a computer (not shown). The motors 76, 77 cause the valve heads 77, 78 to turn by a predetermined angle in accordance with the reaction eluent L as an object to be tested, so that a plurality of flow passages formed in the valve heads 77, 78 can be communicated with the outlet/inlet valves and a feed valve as later described.

That is, the valve heads 77, 78 are provided at their centers with the outlet/inlet valves 79, 80, respectively. Of those valves, the inlet valve 79 is connected with the downstream side end part of the feed eluent tube 25, and the outlet valve 80 is connected with the upstream side end part of the transfer tube 51.

A plurality of feed valves 81, 82 are arranged at the generally equal angular positions on the outside of the outlet/inlet valves 79, 80. Those feed valves 81, 82 are in communication with the outlet/inlet valves 79, 80 through flow passages formed within the valve heads 77, 78.

The outlet/inlet valves 79, 80 and the feed valves 81, 82 are controlled in operation through a computer (not shown) as later described, such that the valves 79 through 82 can be opened and closed.

A plurality of capillary tubes 83 as a reaction tube are connected between the feed valves 81, 82, and the reaction eluent L in the respective tubes 83 are heated to a predetermined temperature all at once so that the target DNA contained in the respective reaction eluent L can be amplified.

That is, a heater 84 is disposed between the valve heads 77, 78. The heater 84 has a hollow cylindrical section, which is stepwise dilated, at an upper part thereof. The capillary tubes 83 are laid in the hollow cylindrical section, and a fan 85 is disposed under the hollow cylindrical section. A heat block 86 is disposed immediately under the fan 85.

The heat block 86 is controlled, by the above-mentioned computer, in heating temperature required for amplifying a target DNA, i.e., in denaturation temperature (about 95 degrees C), annealing temperature (about 60 degrees C) and elongating temperature (about 72 degrees C) for a target DNA, as well as the heating time and the number of times for heating (heating cycle), and the warm air is sent into the heater 84 through the fan 85 so that the capillary tubes 83 can be heated.

One end of the transfer tube 51 is connected to the outlet valve 80, a syringe pump 88 is inserted in the tube 51 through a three-way valve 87, and a multiport switch valve, a 13-port switch valve 90 in this embodiment, is connected to the downstream side of the pump 88 through a three-way valve 89.

The three-way valve 89, when switched, makes it possible to discharge the reaction eluent L, which has been subjected to PCR amplification, to the multiport switch valve 90 or a drain receptacle 91.

The multiport switch valve 90 includes a plurality of outlet/inlet ports which are disposed adjacent to each other and communicated with each other. Of those ports, the inlet ports of the ports P₁, P₄ are connected with one ends of the inlet tubes 92 through 95, and the outlet ports are connected with one ends of the outlet tubes 96 through 99.

The other end parts of the inlet tubes 92 through 95 are laid within a housing 100, and a resistance tube 101 as a fluid resisting means is connected to the inlet tubes 92 through 95 within the housing 100. The resistance tube 101 has the same fluid resistance and its value of resistance is set larger than the pressure of the columns 52a through 52d which are arranged at the DNA detector 5, i.e., fluid resistance (including the fluid resistance of the stationary-phase DNA probe 53 filled therein) 53, approximate two times the pressure of the columns 52a through 52d in this embodiment.

One end of a feed eluent tube 102 is connected to the other ends of the inlet tubes 92 through 95, and the other end of the tube 102 is in communication with an eluent storage receptacle 103. Only one feed eluent pump 104 is inserted in the feed eluent tube 103, so that the eluent in the receptacle 103 can be fed to the inlet tubes 92 through 95.

The columns 52a through 52d are inserted in the downstream parts of the outlet tubes 96 through 99, and the same or different stationary-phase DNA probes 53a through 53d are filled in the columns 52a through 52d or coated onto the inner walls of the columns 52a through 52d.

Heat blocks 56a through 56d are arranged adjacent to the columns 52a through 52d, and those heat blocks 56a through 56d can set the entire area sequentially to a uniform temperature instead of a predetermined gradient formed in the moving direction of the reaction eluent L as previously mentioned.

That is, the heat blocks 56a through 56d controlled by a computer such that the temperature can be increased or decreased. The heat blocks 56a through 56d are heated to about 95 degrees C which is in the vicinity of the temperature for forming a double strand of DNA in the first stage of DNA analysis and thereafter, the heating temperature is reduced to about 40 degrees C which is in the vicinity of the temperature for forming a double strand of DNA. Under such temperature, heat elution is conducted so that the DNA combined with the stationary-phase DNA probe can be detected.

The downstream side end parts of the outlet tubes 96 through 99 are connected to a fraction collector 106 or drain through a multichannel type UV monitor 105 with the computer contained therein, so that the excessive primer, and the like in the reaction eluent L can be separated and collected or discharged to the outside.

The UV monitor 105 calculates the presence/absence of the target nucleic acid, separating position and a peak value based on data preliminarily stored in the computer under the condition of input of the detecting/analyzing signal of the object to be tested, and the result of such calculation is simultaneously displayed in a monitor screen 107 in the form of analysis patterns T₁ through T₄ of SSTEC pattern.

In the monitor screen 107, temperature (degrees C) expressed in the form of retention time is plotted along the abscissa and an absorbance is plotted along the ordinate, so that the analysis patterns T₁ through T₄ of the various objects to be tested are displayed thereon. The peak position in those analysis patterns T1 through T4 indicates the melting temperature (Tm value) of the DNA to be tested.

That is, in this embodiment, a desired quantity of reaction eluent L composed of the same or different kinds of samples generated in the automatic sampler 2 is intermittently sent to the transfer tube 25 through the syringe 15 and this reaction eluent L is sequentially introduced into the inlet valve 79 of the DNA amplifier 4.

In the DNA amplifier 4, the inlet valve 79 and the feed valve 81 are opened based on data stored in the computer under the condition of sending of the reaction eluent L from the automatic sampler 2, and the valve heads 77, 78 are turned to communicate the inlet valve 79 and a predetermined feed valve 91 with each other through various flow passages formed therein: Moreover, the corresponding feed valves 81, 82 are communicated with each other through the capillary tube 83.

Then, the reaction eluent L, which has been sent first, is moved from a predetermined feed valve 82 to the capillary tube 83 through a flow passage formed in the valve head 77 from the inlet valve 79. When the reaction eluent L is moved to the center within the heater 84, it is stopped.

Then, the reaction eluent L, which has been sent second, is moved from a predetermined feed valve 82 to the capillary tube 83 through the flow passage formed in the valve head 77 from the inlet valve 79. When the reaction eluent L is moved to the center within the heater 84, it is stopped.

Thereafter, the reaction eluent L is sequentially moved from the inlet valve 79 to the capillary tube 83 through a predetermined feed valve 82 and when the reaction eluent L is moved to the center within the heater 84, it is stopped.

Then, the various reaction eluents L are sent to the respective capillary tubes 83 and when the reaction eluents L are moved to the central parts, they are stopped. Thereafter, the heat block 86 is heated for a predetermined time until it is heated to a predetermined temperature required for amplifying a target DNA through the computer and the warm air is delivered into the heater 84 through the fan 85 so that the capillary tubes 83 are heated all at once.

That is, the heat block 86 is heated to a temperature (about 95 degrees C) required for denaturating the target DNA first, and the double strands of the DNA contained in the reaction eluents L in the respective tubes 83 are denatured all at once to generate a single strand DNA.

Then, the heat block 86 is reduced in temperature (about 60 degrees C) required for annealing the target DNA, and two kinds of primers are combined with the single strand DNA in the respective reaction eluents L for annealing. Thereafter, the heat block 86 is heated to a temperature (about 72 degrees C) required for elongating the target DNA, and a double strand DNA is synthesized through a heat resisting DNA polymerase to copy a complementary DNA.

In this way, when the basic cycles for analysis in the process for denaturating a target DNA, the annealing process and the elongating process are finished, the heat block 86 is heated again for a predetermined time until it is heated to the denaturating temperature, the annealing temperature and the elongating temperature. Thereafter, the above-mentioned operation is repeated so that a predetermined number of cycles, 30 cycles in this embodiment, are executed, so that the target DNA in the respective reaction eluents L are amplified by a predetermined quantity and then, the PCR amplification process is finished.

In this way, since the reaction eluent L as a sample is stopped at a predetermined position of a specific capillary tube 83, a correct and stable amplification state can be obtained compared with the conventional technique in which the reaction eluent L is moved to a common reaction tube and heated. This is advantageous when different kinds of samples are to be amplified.

Moreover, since a plurality of capillary tubes 83 are heated by only one heat block 86, the structure can be simplified and rationalized, the heat source consumption can be reduced and the amplifying cost can be reduced compared with the conventional technique in which a plurality of heating parts are required.

Moreover, since a plurality of samples are heated to the same temperature and the process for denaturating the DNA of the various samples, the annealing process and the elongating process are executed simultaneously, the time required for amplification can be shortened compared with the conventional technique in which those processes are separately executed.

After the PCR amplification, the outlet valve 80 and a predetermined feed valve 82 are opened based on the data stored in the computer, the respective capillary tubes 83 are communicated with the transfer tube 51 through the respective flow passages formed in the valve heal 78, and the amplified reaction eluents L in the respective capillary tubes 83 are sequentially moved into a predetermined port of the multiport switch valve 90 through the syringe 88.

The multiport switch valve 90 is disposed at an intermediate zone of the passage for feeding the eluent from the feed eluent pump 104 to the DNA detector 5, and the same number of resistance tubes 101 as the objects to be tested are connected in parallel to the upstream side of the feed passage, so that the eluent is flowed separately into the respective resistance tubes 101.

The eluent is moved to the multiport switch valve 90 via the resistance tubes 101. During the movement, the eluent is converged with the amplified reaction eluents L introduced into the port, and they are moved to the stationary-phase DNA probes 53a through 53d of the predetermined columns 52a through 52d through the predetermined outlet tubes 96 through 99.

Thereafter, the amplified reaction eluents L are sequentially introduced into the predetermined ports of the multiport switch valve 90, and the reaction eluents L are moved to the predetermined stationary-phase DNA probes 53a through 53d via the outlet tubes 96 through 99 and the predetermined columns 52a through 52d through the corresponding inlet tubes 92 through 95.

When the same or different kinds of amplified reaction eluents L have been moved to the respective stationary-phase DNA probes 53a through 53d, the respective heat blocks 56a through 56d are heated all at once so that they are heated to the temperature (about 95 degrees C) required for denaturating the target DNA first, so that the double strand of the DNA contained in the reaction eluents L is denatured all at once to generate a single strand DNA.

Thereafter, the heat block 86 is reduced in temperature (hybridization temperature) of about 40 degrees C required for forming a chain, heat eluted under that temperature, the DNA combined with the stationary-phase probes are detected, and then, the heat block 86 is returned to the original state.

In this way, since the entire areas of the respective heat blocks 56a through 56d are increased and decreased in temperature so as to be sequentially set to a uniform temperature in this embodiment, the analysis can be made easily and with an inexpensive equipment compared with the conventional technique in which the heat blocks 56a through 56d form a temperature gradient and then, the DNA to be tested is detected.

In this case, the fluid resistance of the respective tube 101 is set to be approximately equal which is larger than the pressure of the columns 52a through 52d, about 5 times in this embodiment. Accordingly, the quantity of the eluent within the inlet tubes 92 through 95 becomes approximately 1/5 compared with the conventional technique in which the resistance tube 101 is not employed, and variation in flow rate of the columns 52a through 52d is restrained to approximately 1/5 compared with the conventional technique in which the resistance tube 101 is not employed.

That is, owing to a provision of the resistance tube 101, the variation of flow rate of the columns 52a through 52d can be restrained. Accordingly, it is no more required to control the flow rate with precision by disposing an expensive feed eluent pump for each column 52a through 52d.

Therefore, even if a plurality of columns 52a through 52d are arranged in parallel at the DNA detector 5 as in this embodiment, the flow rate can be restrained and this can be coped with only one feed eluent pump 103.

Accordingly, the equipment cost can be reduce to that extent. Moreover, by employing only one feed eluent pump which comparatively frequently gets into trouble, the occurrence of trouble can be prevented and a stable and smooth analysis can be attained.

Moreover, even if the quantity of eluent of the columns 52a through 52d is restrained and reduced in the manner as mentioned above, the retention time of the analytic patterns T₁ through T₄ is temperature dependency and adverse effects due to variation of flow rate can practically be disregarded. Accordingly, correctness and reliability of analysis can be maintained.

Moreover, a plurality of columns 52a through 52d are arranged at the DNA detector 5 so that a plurality of objects to be tested can be treated in parallel. Accordingly, the time required for analysis for each object can be reduced. Thus, a high speed and low cost analysis can be achieved.
In addition, since the analytic patterns T₁ through T₄ of the respective objects to be tested can be displayed, from time to time, in the monitor screen 107, their movements, transitions, changes and differences can easily be obtained.

As an application example of the seventh embodiment, a preheat block 108, as shown in FIG. 15, is faced with outlet tubes 96 through 99 which are disposed between the heat blocks 56a through 56d and the multiport switch valve 90, so that the block 108 can be heated to a predetermined temperature.

In this application mode, the preheat block 108 can be heated to a denaturating temperature (about 95 degrees C) capable of dissociating the double strand of a target DNA and generating a single strand, and the heat blocks 56a through 56d can be heated to a temperature of about 40 degrees C capable for forming a double strand.

By doing so, it can be prevented to control the temperature of the heat blocks 56a through 56d in two ways, so that the temperature setting can easily and simply be made. In addition, by separately heating the heat blocks 56a through 56d and the preheat block 108, the temperature setting can smoothly and surely be made.

The DNA contained in the reaction eluents L is preliminarily made to a single strand before the reaction eluents L are introduced into the heat blocks 56a through 56d. By doing so, only the hybridization reaction is made in the heat blocks 56a through 56d, and the heat elution can be conducted under such temperature. Accordingly, the analyzing time required for increasing and decreasing the temperature can be reduced extensively, and a high speed analysis can be achieved.

### Industrial Applicability

As described hereinbefore, a gene analysis method and an analysis device thereof according to the present invention are suited to be used for a fully automatic gene analysis apparatus or a fully automatic gene diagnostic apparatus.

## Claims

1. A gene analysis method comprising the steps of extracting a target nucleic acid from a biological sample (23) and amplifying a target DNA, thereafter, introducing a reaction eluent (L) containing a predetermined DNA into a stationary-phase DNA probe (53) having a predetermined temperature and arranged in series or in parallel and separating a DNA complementary to said stationary-phase DNA probe (53),
**characterized in that** said gene analysis method comprises a plurality of stationary-phase DNA probes (64 through 66) at least a part of which can be set to a temperature for forming a double strand of a DNA to be tested and a reaction eluent containing the same or different kinds of DNA after being amplified is introduced into said stationary-phase DNA probes (64 through 66).

2. A gene analysis method according to claim 1, wherein said stationary-phase DNA probes (64-66) are increased and decreased in temperature for each analysis of said DNA to be tested.

3. A gene analysis method according to claim 1, wherein said reaction eluent (L) is heated to a predetermined temperature before introduction into said stationary-phase DNA probes (64 through 66) and the double strand of said DNA to be tested is preliminarily denatured so that a single strand is generated.

4. A gene analysis method according to claim 1, wherein a temperature gradient is set such that the upstream side of said stationary-phase DNA probes (64 through 66) with respect to the reaction eluent (L) is a high temperature side and the downstream side of said stationary-phase DNA probes (64 through 66) is a low temperature side.

5. A gene analysis method according to claim 4, wherein a temperature gradient is set such that the upstream side of said stationary-phase DNA probes (64 through 66) is a denaturation temperature or higher of said DNA and the downstream side of said stationary-phase DNA probes (64 through 66) is an extension temperature or lower of said DNA.

6. A gene analysis method according to claim 4, wherein a constant temperature zone where said complementary DNA can form a double strand is provided at an intermediate portion of said temperature distribution so that said stationary-phase DNA probes (64 through 66) are steppingly distributed in temperature.

7. A gene analysis method according to claim 4, wherein a reaction eluent (L) containing the same or different kinds of DNA is directly introduced into said stationary-phase DNA probes (64 through 66).

8. A gene analysis apparatus in which a target nucleic acid is extracted from a biological sample (23), a target DNA is amplified, a reaction eluent (L) containing a predetermined DNA is then introduced into a stationary-phase DNA probe (53) having a predetermined temperature and arranged in series or in parallel and a DNA complementary to said stationary-phase DNA probe (53) is separated,
**characterized in that** said gene analysis apparatus comprises a plurality of stationary-phase DNA probes (64 through 66) at least a part of which can be set to a temperature for forming a double strand of DNA to be tested and a reaction eluent (L) containing the same or different kinds of DNA after being amplified is introduced into said stationary-phase DNA probe (53).

9. A gene analysis apparatus according to claim 8, wherein said stationary-phase DNA probes (64-66) are increased and decreased in temperature for each analysis of DNA to be tested.

10. A gene analysis apparatus according to claim 8, wherein a heat block (108) is provided to a flow passageway of the reaction eluent on the upstream side of said stationary-phase DNA probe, and said heat block (108) can be set to temperatures at which a single strand of DNA can be formed by dissociating the double strand of DNA to be tested.

11. A gene analysis apparatus according to claim 8, wherein a temperature gradient is set such that the upstream side of said stationary-phase DNA probes (64 through 66) with respect to the reaction eluent (L) is a high temperature side and the downstream side of said stationary-phase DNA probes (64 through 66) is a low temperature side.

12. A gene analysis apparatus according to claim 11, wherein a temperature gradient is set such that the upstream side of said stationary-phase DNA probes (64 through 66) is a denaturation temperature or higher of said DNA and the downstream side of said stationary-phase DNA probes (64 through 66) is an extension temperature or lower of said DNA.

13. A gene analysis apparatus according to claim 11, wherein a constant temperature zone where said complementary DNA can form a double strand is provided at an intermediate portion of said temperature distribution so that said stationary-phase DNA probes (64 through 66) are steppingly distributed in temperature.

14. A gene analysis apparatus according to claim 11, wherein a reaction eluent (L) containing the same or different kinds of DNA is directly introduced into said stationary-phase DNA probes (64 through 66).

15. A gene analysis apparatus according to claim 8, wherein a plurality of the same or different stationary-phase DNA probes (64 through 66) are received in a plurality of columns (52a through 52d) which are arranged in parallel with each other and fluid resistance means (101) having a larger fluid resistance than that of said column (52a through 52d) portions is disposed at each supply passageway of an eluent which is in communication with each of said columns (52a through 52d).

16. A gene analysis apparatus according to claim 15, wherein said eluent can be supplied to each column (52a through 52d) through only one feed pump (104).

17. A gene analysis apparatus according to claim 8, wherein a multiport switch valve (90), into which a plurality of reaction eluents (L) containing the same or different kinds of DNA which have been amplified, can be introduced, is disposed between the downstream side of each supply passageway of said eluent and a DNA detection apparatus, and each supply passageway of said eluent and a passageway which is in communication with each of said columns (52a through 52d) are connected to a predetermined port (Pi through P₄) of said switch valve (90).

18. A gene analysis apparatus according to claim 11, wherein a tubular heat block (56) capable of forming said temperature distribution is provided, a light source (54) is disposed at an inner side of said heat block (56), said heat block (56) is provided with transmittance means of said light source (54), and said stationary-phase DNA probes (64 through 66) are arranged in such a manner as to face with said transmittance means.

19. A gene analysis apparatus according to claim 11, wherein only one or a plurality of micro-channels (72) are formed on a base (71) capable of forming said temperature distribution, and only one or a plurality of stationary-phase DNA probes (64 through 66) are arranged within said channels (72).

20. A gene analysis apparatus according to claim 11, wherein an analysis pattern (T₁ through T₄) of each test sample can be displayed in a display screen (107) of an analysis apparatus capable of detecting and analyzing a double strand forming position of the DNA to be tested by said stationary-phase DNA probes (64 through 66).
